Europäisches Patentamt
European Patent Office
Office européen des brevets

(19)

(11) Numéro de publication: **0 165 151**
**A1**

(12)

# DEMANDE DE BREVET EUROPEEN

(21) Numéro de dépôt: **85400997.4**

(22) Date de dépôt: **21.05.85**

(51) Int. Cl.[4]: **C 07 K 5/06**
A 61 K 37/64

(30) Priorité: **25.05.84 FR 8408274**

(43) Date de publication de la demande: **18.12.85 Bulletin 85/51**

(84) Etats contractants désignés: **AT BE CH DE FR GB IT LI LU NL SE**

(71) Demandeur: **SANOFI**
**40, Avenue George V**
**F-75008 Paris(FR)**

(71) Demandeur: **INSTITUT NATIONAL DE LA SANTE ET DE LA RECHERCHE MEDICALE (INSERM)**
**101, rue de Tolbiac**
**F-75654 Paris Cedex 13(FR)**

(72) Inventeur: **Guegan, Rémy**
**355 Chemin du Thym**
**F-34170 Castelnau-Le-Lez(FR)**

(72) Inventeur: **Diaz, Joseph**
**19, rue du Prados**
**F-34470 Perols(FR)**

(72) Inventeur: **Nisato, Dino**
**4 Impasse de l'Olivette**
**F-34680 - St. Georges D'Orques(FR)**

(72) Inventeur: **Corvol, Pierre**
**88, rue de Sèvres**
**F-75007 Paris(FR)**

(74) Mandataire: **Gillard, Marie-Louise et al,**
**Cabinet Beau de Loménie 55, Rue d'Amsterdam**
**F-75008 Paris(FR)**

(54) **Dérivés peptidiques inhibiteurs de la rénine, leur procédé de préparation et leur application en thérapeutique.**

(57) Les composés selon l'invention répondent à la formule générale:

R1 – Phe – R2 – Sta – R3 – Sta – R4 (I)

dans laquelle on désigne par Statyl (Sta) le radical dérivant de l'aminoacide Statine, à savoir:

```
– NH – CH  – CHOH – CH2 – C –
       '                  "
       CH2                O
       '
       CH

 H3C        CH3            isomère (3S, 4S)
```

- R1 représente un groupe protecteur de la fonction amine, tel qu'un groupe alcanoyle et en particulier le groupe isovaléryle, un groupe phénylalcanoyle, un groupe aroyle, un groupe aryl-sulfonyle, un groupe arylakyl-sulfonyle ou encore un groupe t.butoxycarbonyle (BOC), ou benzyloxycarbonyle (Z), ou adamantyloxycarbonyle (Adoc);
- R2 représente le radical dérivé d'un aminoacide choisi parmi la leucine, la norleucine, l'histidine et la norvaline.
- R3 représente le radical dérivé d'un aminoacide choisi parmi l'alanine, la leucine et l'isoleucine.
- R4 est un groupe amino.

EP 0 165 151 A1

Croydon Printing Company Ltd.

Dérivés peptidiques inhibiteurs de la rénine, leur procédé de préparation et leur application en thérapeutique.

La présente invention concerne des nouveaux dérivés peptidiques inhibiteurs de la rénine.

Elle concerne également un procédé pour leur obtention et leur application en thérapeutique.

En 1970, UMEZAWA a isolé, à partir d'une culture de streptomyces, un pentapeptide désigné sous le nom de Pepstatine dont la structure a été établie ultérieurement et répond à la formule :

Isovaléryl - L-valyl - L-valyl - Statyl - L-alanyl - Statine

dans laquelle on désigne sous le nom de statine un acide aminé non usuel, l'acide (3S, 4S) amino-4-hydroxy-3-méthyl-6 heptanoïque.

Il a été montré que la pepstatine est un inhibiteur de protéases acides et agit en particulier sur la pepsine et la rénine. Spécialement, la rénine, enzyme d'origine rénale, intervient dans la séquence angiotensinogène, angiotensine I, angiotensine II au niveau de la transformation de l'angiotensinogène en angiotensine.

L'angiotensine étant responsable de l'élévation de la tension artérielle, on a envisagé d'utiliser la pepstatine pour lutter contre l'hypertension chez l'homme.

Toutefois, la pepstatine agissant sur l'ensemble des protéases acides, son emploi en thérapeutique s'est avéré difficile.

Des dérivés de la pepstatine ont été décrits dans la littérature scientifique. Les plus anciens présentent un niveau d'activité en général modeste et une sélectivité faible. Plus récemment, il est apparu de nouveaux composés peptidiques présentant une activité élevée in vitro en tant qu'inhibiteurs de la rénine. Toutefois, leur activité demeure très limitée in vivo par suite d'une très faible biodisponibilité. Parmi les facteurs pouvant influencer la biodisponibilité, il faut en particulier noter la solubilité dans l'eau qui est très faible pour tous les produits précédemment mentionnés.

La présente invention a pour objet des peptides présentant un niveau élevé d'activité comme inhibiteurs de la rénine tout en possédant une hydrosolubilité accrue.

Les composés selon l'invention répondent à la formule générale :

R1 - Phe - R2 - Sta - R3 - Sta - R4 (I)

dans laquelle on désigne par Statyl (Sta) le radical dérivant de l'aminoacide Statine, à savoir :

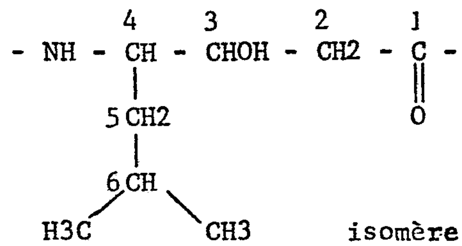

isomère (3S, 4S)

- R1 représente un groupe protecteur de la fonction amine, tel qu'un groupe alcanoyle et en particulier le groupe isovaléryle, un groupe phénylalcanoyle, un groupe aroyle, un groupe arylsulfonyle, un groupe arylalkyl-sulfonyle ou encore un groupe t.butoxycarbonyle (BOC), ou benzyloxycarbonyle (Z), ou adamantyloxycarbonyle (Adoc) ;
- R2 représente le radical dérivé d'un amino acide choisi parmi la leucine, la norleucine, l'histidine et la norvaline.
- R3 représente le radical dérivé d'un aminoacide choisi parmi l'alanine, la leucine et l'isoleucine.
- R4 représente :

- un groupe 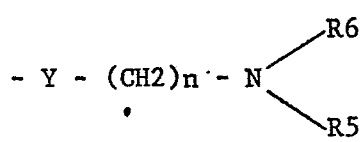

dans lequel : Y est -O-ou -NH-,

n est un entier égal à 2 ou 3,

R5 et R6 pris isolément représentent un groupe alkyle inférieur (1 à 4 C) ou,

R5 et R6 considérés ensemble avec l'atome d'azote auquel ils sont liés forment un cycle, tel que la pyrrolidine, la pipéridine ou la morpholine.

Sont également inclus dans l'invention, les dérivés ammonium quaternaires que peuvent donner les dérivés ci-dessus avec

un composé R7 X où R7 désigne un groupe alkyle inférieur et X l'anion issu d'un acide minéral ou organique.

- un groupe $-Y-(CH_2)_n-SO_3H$
  où Y et n sont tels que définis ci-dessus.
- un groupe $-NH-CH(COOR_8)-(CH_2)_p-R_9$

```
- NH - CH - COOR8
       |
     (CH2)p
       |
       R9
```

dans lequel .R8 désigne l'hydrogène ou un groupe alkyle inférieur,
.p représente un nombre entier égal à 1, 2, 3 ou 4
.R9 désigne un groupe guanidino, amino, $-SO_3H$, ou $-COOR_8$, R8 ayant les significations mentionnées ci-dessus.

Un tel substituant ayant un atome de carbone asymétrique peut exister sous la forme d'isomères optiques D et L ou sous la forme racémique.

- un groupe $-NH-(CH_2)_p-CH(NH_2)-COOR_8$

```
- NH - (CH2)p - CH - COOR8
                |
               NH2
```

où p et R8 sont tels que définis précédemment sous la forme d'isomères optiques ou de racémiques.

Suivant la nature de R4, les composés de formule (I) sont susceptibles de former des sels dérivés d'acides minéraux ou organiques et/ou des sels dérivés de bases minérales ou organiques. Ces sels font partie intégrante de l'invention.

Les produits selon l'invention peuvent être préparés selon les méthodes habituelles de la chimie des peptides.

Ils peuvent, par exemple, être préparés par un procédé étape par étape dit procédé "Stepwise" à partir du C terminal. Le produit de départ est un ester d'alkyle inférieur de l'acide aminé C terminal sur lequel est condensé l'aminoacide suivant de la séquence. Après libération de la fonction amine du dipeptide, on procède à l'élongation de la chaîne peptidique par couplage de l'acide aminé suivant, convenablement protégé.

Chaque phase de couplage est suivie d'une opération sélective de libération de l'amine qui va entrer en réaction lors de la création de la liaison peptidique suivante. Les différentes opérations de couplage sont effectuées, soit en utilisant un ester activé de l'aminoacide à coupler, soit en utilisant l'aminoacide N-protégé en présence de dicyclohexyl carbodiimide.

Les phases de déprotection sélective de l'amine sont effectuées selon la nature du groupe protecteur utilisé, soit par hydrogénolyse, soit par hydrolyse en milieu acide fort tel que l'acide trifluoracétique.

Enfin, lorsque l'acide aminé à introduire dans la séquence possède dans sa chaîne latérale une fonction susceptible de réagir, il convient de bloquer celle-ci par un groupe protecteur convenable que l'on élimine ultérieurement.

Selon ce schéma général, il est possible, soit de préparer l'ensemble de la séquence désirée, soit de préparer 2 fragments de cette séquence que l'on couple finalement pour obtenir le peptide désiré.

Enfin, les peptides (I) dans lesquels R4 ne représente pas un aminoacide, peuvent être obtenus à partir des pentapeptides acides :

R1 - Phe - R2 - Sta - R3 - Sta - OH

par action d'un réactif convenable portant une fonction alcool ou amine selon que l'on souhaite obtenir un ester ou un amide, c'est-à-dire un composé de formule $R_4H$, $R_4$ étant tel que défini ci-dessus.

Les exemples suivants, non limitatifs, sont donnés à titre d'illustration de la présente invention.

Dans ces exemples, les abréviations suivantes seront utilisées :

Acides aminés :

Ces abréviations sont en accord avec celles indiquées par la Commission de Nomenclature de l'IUPAC-IUB, section Biochimie.

Ala : Alanine

Arg : Arginine

Glu : Acide glutamique

Lys : Lysine

Nle : Norleucine

Phe : Phénylalanine

Asp : Acide Aspartique.

Ces acides aminés sont de configuration L.

Groupes protecteurs ou activateurs :

BOC : t. butyloxycarbonyle

OBzl : Ester benzylique

NO2 : Groupe nitro

OMe : Ester méthylique

ONSu : Ester de N-hydroxysuccinimide

iVa : Isovaléryle

OTcp : Ester de trichloro-2,4,5 phényle

Z : Benzoyloxycarbonyle

O But: Ester tertiobutylique.

De plus, les abréviations suivantes sont également utilisées :

AcOEt : Acétate d'éthyle

AcOH : Acide acétique

Bop : Hexafluorophosphate de benzotriazolyl-oxy-tris (diméthylamino) phosphonium

DCCI : Dicyclohexylcarbodiimide

DCU : Dicyclohexylurée

DIPEA : Diisopropyléthylamine

DMF : Diméthylformamide

Ether : Ether éthylique

HOBt : Hydroxy-1 benzotriazole

KHSO4-K2SO4 : Solution aqueuse contenant 16,6 g de bisulfate de potassium et 33,3 g de sulfate de potassium pour 1 litre

MeOH : Méthanol

NEM : N-éthyl-morpholine

TA : Température ambiante

TFA : Acide trifluoracétique

ClNa : Chlorure de sodium

| | |
|---|---|
| CO3HNa | : Bicarbonate de sodium |
| SO4Mg | : Sulfate de magnésium |
| THF | : Tétrahydrofuranne |
| Pd/C | : Palladium sur charbon |
| Fc | : point de fusion |

Enfin, les spectres de résonance magnétique nucléaire ont été enregistrés à 250 MHz en solution dans le diméthylsulfoxyde, l'étalon interne étant l'hexaméthyldisiloxane.

Les abréviations suivantes sont utilisées :

s : singulet

d : doublet

m : multiplet ou massif

t : triplet.

Dans la colonne "Attribution" des tableaux ci-après les chiffres au-dessus des groupements indiquent la position de ceux-ci dans l'acide aminé concerné.

EXEMPLE I :

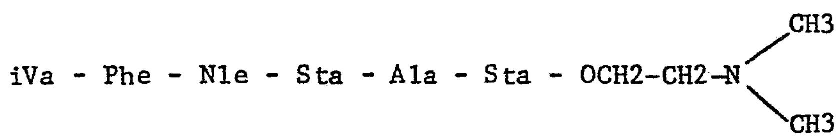

(SR 42764).

1. Z - Ala - Sta - OMe.

Dissoudre à température ambiante dans 35 ml de DMF, 1,44 g de H-Sta-OMe, TFA, 595 mg de NEM, 2,06 g de Z-Ala-OTcp et 1,29 g de HOBt. Ajuster le pH à 6-7 au papier pH, si nécessaire, par NEM et agiter à température ambiante durant 48 h. Evaporer le DMF au bain-marie à 40°C sous 1,33 Pa.

Reprendre l'huile résiduelle dans 50 ml d'AcOEt et laver celui-ci successivement avec KHSO4-K2SO4 5 %, NaCl/H2O, NaHCO3 5 %, NaCl/H2O. Sécher sur MgSO4 et évaporer le solvant.

Reprendre dans l'éther, un solide apparaît. Laisser une heure au réfrigérateur ; essorer ; sécher.

Rendement : 1,46 g (86 %) ; Fc : 117-120°C.

2. H - Ala - Sta - OMe.

Dissoudre 1,46 g de Z-Ala-Sta-OMe dans 20 ml de MeOH, puis, à température ambiante, ajouter 1,03 g de formiate d'ammonium, agiter jusqu'à dissolution, puis ajouter 400 mg de Pd/C 10 % et agiter 5 min à température ambiante. Après 5 min, un contrôle en CCM montre la disparition du produit de départ. Le pH est de 8 au papier pH. Filtrer l'ensemble sur célite et évaporer le solvant. Filtrer le méthanol sur une colonne de résine Amberlite IR 45 (OH) et évaporer le méthanol.

Reprendre dans l'éther et évaporer à sec. Dissoudre dans du chlorure de méthylène, filtrer l'insoluble et évaporer à sec ; on obtient une poudre blanche.

Rendement : 810 mg (75 %) ; Fc : 123-134°C.

3. Boc - Sta - Ala - Sta - OMe.

Dissoudre successivement dans 50 ml de chlorure de méthylène à température ambiante 960 mg de H-Ala-Sta-OMe, 1 g de Boc-Sta-OH, 420 mg de HONSu, 750 mg de DCCI. Très rapidement un précipité apparaît. Agiter à température ambiante. Après 7 h, filtrer la DCU formée et laver celle-ci avec du chlorure de méthylène. Laver la solution organique successivement avec $KHSO_4$-$K_2SO_4$, (5 % $H_2O$), $NaHCO_3$ 5 % $H_2O$. Sécher sur $MgSO_4$, filtrer et évaporer le solvant. Le produit est solubilisé dans le minimum de mélange chloroforme-MeOH 97,5-2,5 et déposé en tête d'une colonne (L : 24 cm, diamètre : 2,5 cm) de gel de silice Merck H R type 60, montée dans le mélange. Eluer avec le même mélange et fractionner.

On obtient un lot de produit pur (rendement : 1 g) et deux lots de produit à recycler dans les mêmes conditions, d'où le rendement global :

Rendement : 67 % ; Fc : 95-8°C.

4. H - Sta - Ala - Sta - OMe, TFA.

1 g de Boc-Ala-Sta-OMe est solubilisé dans 5 ml de chlorure de méthylène. Ajouter 10 ml de TFA à température ambiante et laisser ainsi 30 min. Evaporer les solvants sous trompe à eau. Reprendre l'huile résiduelle dans l'éther. Essorer le solide obtenu ; rincer à l'éther ; sécher.

Rendement : 850 mg (85 %) ; Fc : 148-151°C.

5. Boc - Nle - Sta - Ala - Sta - OMe.

531 mg de H-Sta-Ala-Sta-OMe, TFA, 393 mg de Boc-Nle-ONSu et 162 mg de HOBt sont recouverts de 5 ml de dioxane contenant 391 mg de NEM. Ajuster le pH à 6-7 par NEM si nécessaire et agiter 48 h à TA. Evaporer le solvant, dissoudre le résidu dans ACOEt et laver successivement par KHSO4-K2SO4 5 %, eau/ClNa, NaHCO3 5 %, eau/ClNa. Sécher sur MgSO4, évaporer le solvant. Dissoudre le résidu dans l'éther et précipiter le produit par le pentane ; essorer ; sécher.

Rendement : 570 mg (90 %).

6. H - Nle - Sta - Ala - Sta - OMe, TFA.

570 mg de Boc-Nle-Sta-Ala-Sta-OMe sont recouverts de 5 ml de TFA. Après 15 min à TA, évaporer le solvant et reprendre le résidu dans un mélange éther-hexane 1/1. Essorer le solide, sécher sous vide.

Rendement : 570 mg (100 %).

7. iVa - Phe - Nle - Sta - Ala - Sta - OMe.

Dans 3 ml de dioxane contenant 89 mg de NEM, on ajoute 150 mg de H-Nle-Sta-Ala-Sta-OMe, TFA, 36 mg de HOBt et 94 mg de iVa-Phe-ONSu. Agiter à TA. Ajuster le pH à 6-7 par NEM si nécessaire. Après 24 h, essorer le solide, laver au dioxane puis à l'éther, sécher.

Rendement : 130 mg (73 %).

Analyse d'acides aminés : Ala : 1,02 (1) - Nle : 1,01 (1) - Sta : 1,95 (2) - Phe : 1,01 (1).

8. iVa - Phe - Nle - Sta - Ala - Sta - OH.

100 mg de iVa-Phe-Nle-Sta-Ala-Sta-OMe sont solubilisés dans 10 ml de DMF à TA. Ajouter 1 ml d'eau distillée, puis à TA, 0,26 ml de soude normale (2 équivalents). Agiter 30 min à TA puis ajouter 0,26 ml d'acide chlorhydrique normal, le pH est alors de 5. Evaporer les solvants, reprendre le résidu dans l'eau : essorer le solide, laver à l'eau, puis à l'éther ; sécher.

Rendement 75 mg.

Analyse d'acides aminés : Ala : 0,99 (1) - Nle : 1,00 (1) - Sta : 2,04 (2) - Phe : 0,98 (1).

CH3

9. iVa-Phe-Nle-Sta-Ala-Sta-OCH2—CH2-N

CH3

Dans 10 ml de dioxane contenant 85 mg de N-diméthyl amino-2 éthanol, on ajoute successivement 225 mg de iVa-Phe-Nle-Sta-Ala-Sta-OH et 162 mg de BOP. Agiter à TA ; après une heure, ajouter 2,5 ml de DMF, puis agiter 24 heures. Evaporer les solvants sous haut vide. Reprendre le résidu dans l'eau, et le solide obtenu est essoré, lavé à l'eau, puis avec NaHCO3 5 %, et à l'eau. Laver à l'éther ; sécher.

Rendement : 210 mg (92 %).

Analyse d'acides aminés : Ala : 0,95 (1) - Nle : 1,05 (1) - Phe : 1,00 (1) - Sta : 1,95 (2).

Spectre de RMN du composé SR 42764

| Delta ppm | Aspects | Intégration | Attribution |
|---|---|---|---|
| 0,53-0,95 | m | 21 H | 6<br>-C(CH3)2 Sta<br>-CH3 Nle<br>3<br>-C(CH3)2 iVa |
| 0,95-1,93 | m | 18 H | -CH3 Ala<br>5 6<br>-CH2 - CH Sta<br>2<br>-CH2 - CH iVa<br>-CH2-CH2-CH2-Nle |
| 1,93-2,55 | m | 12 H | (CH3)2 N-<br>2<br>-CH2- Sta<br>-CH2 N< |
| 2,58-3,02 | m | 2 H | 2<br>-CH2- Phe |
| 3,70-3,80 | m | 4 H | 4 3<br>-CH - CH - Sta |
| 3,98-4,01 | t | 2 H | - O CH2 - |
| 4,13-4,30 | m | 2 H | 2<br>-CH< Nle<br>2<br>-CH< Ala |
| 4,45-4,60 | m | 1 H | 2<br>-CH Phe |
| 4,83-5,95 | m | 2 H | -OH Sta |
| 7,08-7,33 | m | 5 H | Aromatiques Phe |
| 7,33-7,53 | m | 2 H | -NH- |
| 7,81-7,95 | d | 1 H | -NH- |
| 7,98-8,08 | m | 2 H | -NH- |

EXEMPLE II :

iVa - Phe - Nle - Sta - Ala - Sta - O - CH2-CH2-$N^{+}(CH_3)_3$ $I^{-}$
(SR 42764 A).

75 mg de iVa- Phe-Nle-Sta-Ala-Sta-O-CH2-CH2-N(CH3)2 (Exemple I-9) sont solubilisés dans 5 ml de MeOH. Ajouter 28,5 mg d'iodure de méthyle. Agiter 5 min à TA et abandonner une nuit au réfrigérateur. Evaporer le solvant après avoir filtré, et reprendre le résidu dans l'éther. Triturer, essorer, sécher.

Rendement : 75 mg (84 %).

Analyse d'acides aminés : Ala : 1,02 (1) - Nle : 1,06 (1) - Phe : 1,00 (1) - Sta : 1,94 (2).

Spectre de RMN du composé SR 42764 A

| Delta ppm | Aspects | Intégration | Attribution |
|---|---|---|---|
| 0,52-0,90 | m | 21 H | 6<br>-C-(CH3)2 Sta<br>-CH3 Nle<br>3<br>-C (CH3)2 iVa |
| 1,08-1,86 | m | 18 H | - CH3 Ala<br>5 6<br>-CH2- CH- Sta<br>2 3<br>-CH2- CH- iVa<br>-CH2-CH2-CH2 Nle |
| 1,98-2,40 | m | 6 H | 2<br>-CH2- Sta<br>-CH2-N |
| 2,58-3,00 | m | 2 H | 2<br>-CH2 Phe |
| 3,06 | s | 9 H | -N (CH3)3 |
| 3,65-3,91 | m | 6 H | 4 3<br>- CH - CH - Sta<br>-O CH2- |
| 4,11-4,26 | m | 2 H | 2<br>-CH< Nle<br>2<br>-CH< Ala |
| 4,40-4,57 | m | 1 H | 2<br>-CH< Phe |
| 7,06-7,24 | m | 5 H | Aromatiques Phe |
| 7,32-7,44 | m | 2 H | -NH- |
| 7,85-7,88 | d | 1 H | -NH- |
| 7,96-8,06 | m | 2 H | -NH- |

EXEMPLE III :

iVa - Phe - Nle - Sta - Ala - Sta - NH - $CH_2$–$CH_2$–N($CH_3$)($CH_3$)
(SR 42 762).

Dans 10 ml de dioxane contenant 112 mg de diméthylamino-2-éthylamine, on ajoute 350 mg de iVa-Phe-Nle-Sta-Ala-Sta-OH (Exemple I-8) et 215 mg de BOP. Agiter à TA, puis ajouter 15 ml de DMF. Après 24 h, évaporer les solvants sous haut vide au bain-marie à 40°C. Reprendre l'huile résiduelle dans l'eau et le produit vitreux obtenu est essoré, lavé à l'eau, puis avec $NaHCO_3$ 5 %, et à l'eau, sécher.

Rendement : 290 mg (88 %).

Analyse d'acides aminés : Ala : 0,96 (1) - Nle : 1,04 (1) - Phe : 1,00 (1).

Spectre de RMN du composé SR 42762

| Delta ppm | Aspects | Intégration | Attribution |
|---|---|---|---|
| 0,53-0,95 | m | 21 H | 6<br>-C $(CH_3)_2$ Sta<br>$-CH_3$ Nle<br>3<br>-C $(CH_3)_2$ iVa |
| 1,08-1,88 | m | 18 H | $-CH_3$ Ala<br>5 6<br>$-CH_2$ - CH- Sta<br>2 3<br>$-CH_2$ - CH- iVa<br>- $(CH_2)_3$ Nle |
| 1,91-2,16 | m | 10 H | 2<br>- $CH_2$ Sta<br>- N($CH_3$)($CH_3$) |
| 2,16-2,30 | t | 2 H | - $CH_2$ N |
| 2,58-3,00 | m | 2 H | 3<br>$-CH_2-$ Phe |
| 3,00-3,16 | m | 2 H | - NH - $CH_2$ |
| 3,66-3,83 | m | 4 H | 4 3<br>-CH - CH- Sta |
| 4,11-4,28 | m | 2 H | 2<br>-CH< Nle<br>2<br>-CH< Ala |
| 4,50-4,58 | m | 1 H | 2<br>-CH Phe |
| 4,83-4,96 | m | 2 H | -OH Sta |

| | | | |
|---|---|---|---|
| 7,08-7,2 | m | 5 H | Aromatiques Phe |
| 7,33-7,45 | m | 2 H | -NH- |
| 7,61 | t | 1 H | -NH - CH2 |
| 7,93 | d | 1 H | -NH- |
| 7,96-8,08 | m | 2 H | -NH- |

EXEMPLE IV :

iVa - Phe - Nle - Sta - Ala - Sta - NH - CH2-CH2 - 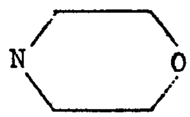

(SR 42 761).

On opère comme dans l'exemple III en remplaçant la diméthylamino-2 éthylamine par une quantité équivalente de morpholino-2 éthylamine.

Spectre de RMN du composé SR 42761

| Delta ppm | Aspects | Intégration | Attribution |
|---|---|---|---|
| 0,57-0,86 | m | 21 H | 6<br>-C (CH3)2 Sta<br>- CH3 Nle<br>3<br>-C (CH3)2 iVa |
| 0,95-1,88 | m | 18 H | - CH3 Ala<br>5 6<br>-CH2 - CH Sta<br>2 3<br>-CH2 - CH iVa<br>- (CH2)3 Nle |
| 1,88-2,10 | m | 4 H | 2<br>-CH2- Sta |
| 2,10-2,40 | m | 6 H | CH2-<br>- CH2 - N<br>CH2- |
| 2,58-3,00 | m | 2 H | 2<br>-CH2- Phe |
| 3,00-3,18 | m | 2 H | - NH - CH2 - |
| 3,45-3,50 | m | 4 H | - CH2<br>O<br>- CH2 |
| 3,67-3,88 | m | 4 H | 4 3<br>-CH - CH- Sta |

| | | | |
|---|---|---|---|
| 4,13-4,27 | m | 2 H | 2 -CH< Nle<br>2 -CH< Ala |
| 4,44-4,59 | m | 1 H | 2 -CH< Phe |
| 4,80-4,90 | m | 2 H | -OH Sta |
| 7,04-7,22 | m | 5 H | Aromatiques Phe |
| 7,27-7,44 | m | 2 H | -NH- |
| 7,57-7,68 | t | 1 H | - NH - CH2 |
| 7,86-7,91 | d | 1 H | -NH- |
| 7,95-8,03 | m | 2 H | -NH- |

EXEMPLE V :

iVa - Phe - Nle - Sta - Ala - Sta - Glu - OH. (SR 42 763).

1) - iVa - Phe - Nle - Sta - Ala - Sta - Glu(OMe)OMe.

301 mg de iVa-Phe-Nle-Sta-Ala-Sta-OH sont solubilisés dans 10 ml de DMF contenant 144 mg de DIPEA ; ajouter 84,5 mg de H-Glu(OMe)OMe, HCl et 215 mg de BOP. Agiter à TA et ajuster le pH à 6-7 par DIPEA en cours de réaction. Après 24 heures, évaporer le solvant au bain-marie à 40°C sous haut vide. Reprendre le résidu dans l'eau, triturer, essorer, laver à l'eau, sécher.

Rendement : 330 mg (91 %).

2) - iVa - Phe - Nle - Sta - Ala - Sta - Glu - OH.

90 mg de iVa-Nle-Sta-Ala-Sta-Glu(OMe)OMe sont solubilisés dans 2 ml de DMF à TA ; ajouter 0,3 ml de soude normale et agiter 1 heure à TA. Ajouter alors 0,3 ml d'acide chlorhydrique normal et évaporer les solvants à sec. Triturer le résidu dans l'eau et essorer. Laver à l'eau et essorer. Dissoudre dans MeOH et évaporer à sec, puis renouveler l'opération. Enfin, dissoudre dans MeOH et précipiter le produit par l'éther ; essorer ; sécher.

Rendement : 65 mg (74 %).

Analyse d'acides aminés : Ala : 1,01 (1) - Nle : 1,02 (1) - Phe : 0,99 (1).

Spectre de RMN du composé SR 42763

| Delta ppm | Aspects | Intégration | Attribution | |
|---|---|---|---|---|
| 0,60-0,88 | m | 21 H | 6<br>-C (CH3)2<br>- CH3<br>3<br>-C (CH3)2 | <br>Sta<br>Nle<br><br>iVa |
| 1.01-1,90 | m | 20 H | - CH3<br>5 6<br>-CH2 - CH-<br>2 3<br>- CH2 - CH-<br>- (CH2)3<br>3<br>- CH2 - | Ala<br><br>Sta<br><br>iVa<br>Nle<br><br>Glu |
| 1,90-2,30 | m | 6 H | 2<br>- CH2 -<br>4<br>- CH2 - | <br>Sta<br><br>Glu |
| 2,58-3,00 | m | 2H | 3<br>-CH2- | <br>Phe |
| 3,58-3,88 | m | 4 H | 4 3<br>-CH - CH - | <br>Sta |
| 4,00-4,30 | m | 3 H | 2<br>-CH<<br>2<br>-CH<<br>2<br>-CH< | <br>Ala<br><br>Glu<br><br>Nle |
| 4,43-4,58 | m | 1 H | 2<br>-CH< | <br>Phe |
| 4,60-5,15 | m | 2 H | -OH | Sta |
| 7,00-7,26 | m | 5 H | Aromatiques | Phe |
| 7,26-7,48 | m | 2 H | -NH- | |
| 7,76-7,93 | m | 2 H | -NH- | |
| 7,95-8,06 | m | 2 H | -NH- | |

EXEMPLE VI :

iVa - Phe - Nle - Sta - Ala - Sta - Arg - OMe. (SR 42 406).

A) - Fragment iVa - Phe - Nle - Sta - OH.

1 - Boc - Nle - Sta - OMe.

Dans 40 ml de dioxane contenant 1,058 g de NEM, on ajoute successivement 912 mg de H-Sta-OMe, TFA, 1,03 g de Boc-Nle-ONSu et 418 mg de HOBt. Agiter 24 heures, évaporer à TA et ajuster le pH à 6-7 par NEM si nécessaire. Après 24 heures, évaporer le solvant, dissoudre l'huile résiduelle dans AcOEt et laver la phase organique successivement par KHSO4-K2SO4 5 %, ClNa-eau, NaHCO3 5 %, ClNa-eau. Sécher sur MgSO4, évaporer le solvant. Reprendre dans un minimum d'éther, ajouter du pentane, essorer le solide ; sécher.

Rendement : 960 mg (80 %).

2 - H - Nle - Sta - OMe, TFA.

800 mg de Boc-Nle-Sta-OMe sont recouverts de 8 ml de TFA à TA. Après 30 minutes, évaporer le solvant à fond : nous obtenons une huile qui est utilisée aussitôt.

3 - iVa - Phe - Nle - Sta - OMe.

H-Nle-Sta-OMe, TFA sous forme d'huile obtenu ci-dessus est solubilisé dans 20 ml de dioxane. Ajouter 736 mg de NEM, 297 mg de HOBt et 871 mg de iVa-Phe-ONSu. Agiter à TA et ajuster le pH à 6-7 par NEM si nécessaire. Après 24 heures, évaporer le solvant, reprendre le résidu dans AcOEt, essorer, laver avec AcOEt, éther ; sécher.

Rendement : 700 mg (70 %).

4 - iVa - Phe - Nle - Sta - OH.

533 mg de iVa-Phe-Nle-Sta-OMe sont solubilisés dans 20 ml de DMF à TA. Ajouter ensuite 2 ml de soude normale et agiter 30 minutes à TA. Ajouter ensuite 2 ml d'acide chlorhydrique normal : le pH est alors 5 au papier pH. Evaporer les solvants sous haut vide, reprendre le résidu dans l'eau, essorer, laver à l'eau, puis à l'éther. Dissoudre le solide dans MeOH, filtrer, évaporer le solvant, reprendre le résidu dans l'éther, triturer, essorer ; sécher.

Rendement : 450 mg (86 %).

B) - Fragment Ala - Sta - Arg - OMe.

1 - Boc - Sta - Arg(NO2) - OMe.

Dans 50 ml de DMF contenant 460 mg de NEM, on ajoute 1,07 g de H-Arg(NO2)OMe commerciale. Agiter jusqu'à solubilisation du solide, puis ajouter 1,1 g de Boc-Sta-OH et agiter jusqu'à solubilisation ; ajouter alors 824 mg de DCCI et agiter une nuit à TA. Refroidir et filtrer la DCU, évaporer le DMF sous haut vide au bain-marie à 40°C et reprendre le résidu dans AcOEt, agiter et refroidir : filtrer la DCU, puis laver la solution organique successivement par KHSO4-K2SO4 5 %, eau/ClNa, NaHCO3 5 %, eau/ClNa. Sécher sur MgSO4 et évaporer le solvant.

Dissoudre le résidu dans MeOH-chloroforme (1-99) et déposer en tête d'une colonne (L : 45 cm - diam. : 1,5 cm) de gel de silice 60 Merck (70-230 mesh) dans le même mélange solvant.

Eluer avec :
. 100 ml MeOH-chloroforme 1-99
. 100 ml MeOH-chloroforme 2-98
. 100 ml MeOH-chloroforme 4-96
. 300 ml MeOH-chloroforme 5-95.

Fractionner ; évaporer les fractions contenant le produit pur. Reprendre dans l'éther ; essorer ; sécher.

Rendement : 520 mg (26 %).

2 - Z - Ala - Sta - Arg(NO2)OMe.

490 mg de Boc-Sta-Arg(NO2)OMe sont recouverts de 4 ml de TFA. Après 15 min à TA, évaporer le solvant, reprendre le résidu dans l'éther : on obtient un solide blanc qui est essoré, lavé à l'éther et séché sous vide. Dissoudre le solide dans 20 ml de DMF

et amener à pH 7-8 par NEM, puis ajouter 352 mg de Z-Ala-ONSu et 148 mg de HOBt. Agiter à TA et ajuster le pH à 6-7 par NEM si nécessaire. Après une nuit, évaporer les solvants sous haut vide, dissoudre l'huile résiduelle dans AcOEt et laver successivement par $KHSO_4$-$K_2SO_4$ 5 %, eau/ClNa, $NaHCO_3$ 5 %, eau/ClNa. Sécher sur $MgSO_4$ et évaporer le solvant. Dissoudre dans AcOEt et déposer en tête de colonne (L . 45 cm - diam. : 1,5 cm) de gel de silice 60 Merck dans AcOEt. Eluer avec AcOEt ; fractionner.

Rendement : 400 mg (66 %).

3 - H - Ala - Sta - Arg - OMe, 2 H Cl.

400 mg de Z-Ala-Sta-Arg ($NO_2$) OMe sont solubilisés dans 10 ml de THF ; ajouter 10 ml d'eau distillée et 1,95 ml d'acide chlorhydrique normal (3 équivalents) et 50 mg de Pd/C 5 %. Hydrogéner sous pression d'une colonne de 1 mètre d'eau. Après 48 heures, la réaction est terminée; filtrer sur célite, bien laver celle-ci par MeOH et évaporer le solvant : nous obtenons une huile utilisée telle quelle.

C - <u>iVa - Phe - Nle - Sta - Ala - Sta - Arg - OMe</u>.

H-Ala-Sta-Arg-OMe, 2H Cl sous forme d'huile préparé ci-dessus est solubilisé dans 25 ml de DMF ; amener à pH 8 par DIPEA, puis ajouter 342 mg de iVa-Phe-Nle-Sta-OH et 353 mg de BOP. Agiter à TA et ajuster le pH à 6-7 par DIPEA en cours de réaction. Après une nuit, évaporer les solvants sous haut vide, puis dissoudre le produit dans MeOH et ajouter 5 ml de gel de silice 60 Merck (70-230 mesh) puis évaporer le solvant à sec. Déposer le solide en tête d'une colonne (L : 40 cm - diam. : 1,5 cm) de même silice dans MeOH-chloroforme 5-95.

Eluer avec : . 280 ml MeOH-chloroforme 5-95
. 400 ml MeOH-chloroforme 10-90
. 600 ml MeOH-chloroforme 15-85.

Fractionner ; évaporer les fractions contenant le produit pur. Reprendre dans l'éther ; essorer et sécher.

Rendement : 170 mg (35 %).

CCM : chloroforme-MeOH-AcOH 80-15-5.

Rf = 0,19.

Spectre de RMN du composé SR 42406

| Delta ppm | Aspects | Intégration | Attribution |
|---|---|---|---|
| 0,59-0,90 | m | 21 H | 6<br>- C (CH3)2 Sta<br>- CH3 Nle<br>3<br>-C (CH3)2 iVa |
| 1,11-1,95 | m | 22 H | - CH3 Ala<br>5 6<br>-CH2 - CH- Sta<br>2 3<br>-CH2 - CH- iVa<br>- (CH2)3 Nle<br>3 4<br>-CH2 - CH2- Arg |
| 2,00-2,24 | m | 4 H | 2<br>- CH2 - Sta |
| 2,60-3,16 | m | 4 H | 3<br>- CH2 Phe<br>5<br>-CH2- Arg |
| 3,59 | s | 3 H | -COO CH3 |
| 3,72-3,85 | m | 4 H | 4 3<br>- CH - CH - Sta |
| 4,14-4,31 | m | 3 H | 2<br>- CH< Nle<br>2<br>- CH< Ala<br>2<br>- CH< Arg |
| 4,45-4,77 | m | 1 H | 2<br>- CH< Phe |
| 4,78 | d | 1 H | -OH Sta |
| 4,85 | d | 1 H | -OH Sta |
| 6,68-7,52 | m | 10 H | Aromatiques Phe<br>NH2<br>- C<br>NH<br>2-NH- |
| 7,52-7,67 | t | 1 H | 6<br>- NH - Arg |
| 7,88 | d | 1 H | -NH- |
| 7,93-8,04 | m | 1 H | -NH- |
| 8,23 | d | 1 H | -NH- |

EXEMPLE VII :

iVa - Phe - Nle - Sta - Ala - Sta - Lys - OH (SR 42 654).

A) - Fragment Boc - Ala - Sta - Lys (Z) OBzl (4 NO2).

1 - Boc - Sta - Lys (Z) OBzl (4 NO2).

1,3 g de Boc-Lys (Z) OBzl (4 NO2) sont recouverts de 10 ml de TFA à TA. Après 30 min, évaporer le solvant à fond. Solubiliser l'huile résiduelle dans 40 ml de AcOEt et amener à pH 8 par NEM ; puis ajouter 550 ml de Boc-Sta-OH et, après solubilisation, ajouter 412 mg de DCCI. Agiter 24 h à TA, refroidir, filtrer la DCU et laver la solution organique successivement par KHSO4-K2SO4 5 %, eau/ClNa, NaHCO3 5 %, eau/ClNa. Sécher sur MgSO4. Evaporer le solvant. Dissoudre le résidu dans 5 ml de chloroforme et déposer en tête d'une colonne (L : 45 cm - diam. : 1,5 cm) de gel de silice 60 Merck (70-230 mesh).

Eluer avec :
- . 100 ml de chloroforme
- . 100 ml MeOH-chloroforme 1-99
- . 100 ml MeOH-chloroforme 2-98
- . 300 ml MeOH-chloroforme 3-97.

Fractionner. Evaporer les fractions contenant le produit pur. Dissoudre dans l'éther, évaporer. On obtient une mousse semi-solide.

Rendement : 730 mg (54 %).

2 - H - Sta - Lys - (Z) OBzl (4 NO2).

720 mg de Boc-Sta-Lys (Z) OBzl (4 NO2) sont recouverts de 6 ml de TFA à TA. Après 15 min, évaporer le solvant, on obtient une huile qui est utilisée ainsi.

3 - Boc - Ala - Sta - Lys (Z) OBzl (4 NO2).

Le sel de TFA de H-Sta-Lys - OBzl (4 NO2) sous forme d'huile préparé ci-dessus, est solubilisé dans 25 ml d'AcOEt et amené à pH 8 par NEM. Ajouter 158 mg de HOBt et 235 mg de Boc-Ala-ONSu. Agiter à TA et ajuster le pH à 6-7 par NEM si nécessaire. Après 48 h, laver la solution organique successivement par KHSO4-K2SO4 5 %, eau/ClNa, NaHCO3 5 %, eau/ClNa. Sécher sur MgSO4, évaporer le solvant. Dissoudre le produit dans 4 ml de

chloroforme et déposer en tête d'une colonne (L : 45 cm - diamètre 1,5 cm) de gel de silice 60 Merck (70-230 mesh) dans le chloroforme.

Eluer avec : . 400 ml de chloroforme
. 800 ml MeOH-chloroforme 1-99.

Fractionner. Evaporer les fractions contenant le produit pur. Reprendre le résidu dans l'éther, évaporer.

Rendement : 310 mg (39 %).

B) - SR 42 654.

1 - iVa - Phe - Sta - Ala - Lys (Z) OBzl (4 NO2).

300 mg de Boc-Ala-Sta-Lys (Z) OBzl (4 NO2) sont solubilisés dans 10 ml de chlorure de méthylène ; ajouter 5 ml de TFA et laisser 30 min à TA. Evaporer les solvants, reprendre le résidu dans l'éther et évaporer celui-ci. L'huile obtenue est solubilisée dans 10 ml de dioxane et le pH amené à 8 par DIPEA. Ajouter alors 207 mg de iVa-Phe-Nle-Sta-OH (Exemple VI A) et 215 mg de BOP. Agiter et ajuster le pH à 6-7 par DIPEA. Après 24 h, essorer le précipité, laver avec AcOEt, éther ; sécher.

Rendement : 300 mg (67 %).

2 - iVa - Phe - Nle - Sta - Ala - Sta - Lys - OH

115 mg de iVa-Phe-Nle-Sta-Ala-Sta-Lys (Z) OBzl (4 NO2) sont solubilisés dans un mélange de 5 ml de DMF et 30 ml MeOH. Ajouter 20 mg de Pd/C 5 % et hydrogéner sous 1 m de colonne d'eau. Après 24 h, filtrer sur célite, laver la célite au DMF, puis avec MeOH. Evaporer les solvants. Dissoudre le résidu dans MeOH, filtrer, évaporer à sec. Reprendre dans l'éther, évaporer à sec.

Rendement : 30 mg (34 %).

Spectre de RMN du composé SR 42654

| Delta ppm | Aspects | Intégration | Attribution |
|---|---|---|---|
| 0,53-0,90 | m | 21 H | 6<br>- C (CH3)2 Sta<br>- CH3 Nle<br>3<br>-C (CH3)2 iVa |
| 1,05-1,92 | m | 24 H | -CH3 Ala<br>5 6<br>- CH2 - CH - Sta<br>2 3<br>- CH2 - CH- iVa<br>- (CH2)3 - Nle<br>3,4,5<br>-(CH2)3- Lys |
| 1,95-2,26 | m | 4 H | 2<br>- CH2 - Sta |
| 2,50-4,60 | m | Perturbé par DOH | 3<br>- CH2 - Phe<br>6<br>- CH2 - Lys<br>4 3<br>- CH - CH- Sta<br>2<br>- CH< Phe, Nle<br>Ala, Lys |
| 7,00-7,22 | m | 5 H | Aromatiques Phe |
| 7,35-8,25 | m | 5 H | -NH- |

EXEMPLE VIII :

iVa-Phe-Nle-Sta-Ala-Sta-Asp-OH, Disel de trishydroxyméthyl-aminométhane (SR 43504 B).

1) iVa-Phe-Nle-Sta-Ala-Sta-Asp(O But)-O But.

Dans 20 ml de DMF contenant 167 mg de DIPEA, on ajoute 301 mg de pentapeptide iVa-Phe-Nle-Sta-Ala-Sta-OH (exemple 1-8), 128 mg de H-Asp-(O But)-O But, HCl et 215 mg de Bop. On agite à température ambiante en ajustant le pH à 6-7 par addition de DIPEA. On laisse 15 heures à température ambiante puis on évapore sous vide poussé en ne dépassant pas la température de 35°C. On reprend le résidu dans l'eau et essore le solide. On le lave successivement avec KHSO4-K2SO4, avec de l'eau, avec une solution de bicarbonate à 5 % puis avec de l'eau et enfin avec de l'éther.

Après séchage, on obtient 310 mg du produit attendu.

Rendement 79 %.

Analyse d'acides aminés :

Asp : 0,94 (1) - Ala : 0,99 (1) - Nle : 1,03 (1) - Phe : 1,05 (1) - Sta : 2,01 (2).

2) iVa-Phe-Nle-Sta-Ala-Sta-Asp-OH.

A 200 mg du produit obtenu ci-dessus, on ajoute 2 ml de TFA et laisse 1 heure 30 à température ambiante. On évapore sous vide et reprend le résidu dans l'éther. On essore le solide, lave avec de l'éther et sèche.

Poids : 170 mg ; Rendement : 98 %.

3) Disel de trishydroxyméthyl-aminométhane (TRIS)

A 172 mg du peptide préparé ci-dessus en suspension dans 30 ml d'éthanol absolu, on ajoute la solution de 48,4 mg de TRIS dans 10 ml d'eau. On agite à température ambiante jusqu'à dissolution totale et poursuit l'agitation encore 30 minutes. On évapore les solvants sous vide. On reprend le résidu dans l'éthanol et évapore à nouveau.

On reprend le résidu dans l'éther et essore le solide formé. Après séchage, on obtient 210 mg du disel attendu.

Rendement : 95 %.

Analyse d'acides aminés :

Asp : 0,92 (1) - Ala : 1,00 (1) - Nle : 1,02 (1) - Phe : 0,98 (1) - Sta : 1,99 (2) - H2N-C-(CH2OH)3 : 2,02 (2).

Les produits selon l'invention ont été étudiés en ce qui concerne leurs propriétés thérapeutiques et notamment leur action inhibitrice enzymatique. Plus particulièrement, les composés ont été évalués "in vitro", sur l'inhibition de l'activité Rénine Plasmatique Humaine.

I - METHODES :

Inhibition de l'activité Rénine Plasmatique Humaine (A.R.P.).

La méthode utilisée est inspirée de GUYENE (J. Clin. Endocri. Metab. 43, p. 1301, 1976) dans la mesure où l'inhibition de l'A.R.P. est évaluée à partir d'un pool de plasmas humains, riche en rénine (15 à 20 mg d'angiotensine I libérée par millitre et par heure) incubé à 37°C en présence de concentrationscroissantes du produit à étudier.

L'angiotensine I libérée au cours de la réaction (les plasmas humains contiennent et le substrat : l'angiotensinogène, et l'enzyme : la rénine) est mesurée par dosage radio-immunologique à l'aide d'un kit.

Bien entendu, un inhibiteur de l'enzyme de conversion du PMSF (phénylméthylsulfonyl fluorure) est rajouté au milieu d'incubation.

Les expériences ont été effectuées à pH 7,4 qui est le pH physiologique (tampon phosphate, incubation 60 minutes).

Les résultats sont exprimés par la dose de composé évaluée en moles qui inhibe de 50 % (IC 50) l'activité Rénine Plasmatique Humaine présente en l'absence d'inhibiteur.

II - RESULTATS :

Les résultats obtenus avec divers produits de l'invention sont représentés dans le tableau suivant où figurent les IC 50 de chaque molécule en ce qui concerne leur inhibition de l'activité rénine plasmatique humaine. De 5 à 10 doses ont été nécessaires pour déterminer ces IC 50. La pepstatine, en tant que substance de référence, est toujours testée en parallèle dans chaque expérience.

TABLEAU I

| Numéro de Code | Inhibition de l'A.R.P. humaine à pH 7,4 IC 50 M |
|---|---|
| Pepstatine | $1,2$ à $1,4 \times 10^{-5}$ |
| SR 42 406 | $2,7 \times 10^{-8}$ |
| SR 42 654 | $5,6 \times 10^{-8}$ |
| SR 42 761 | $1,3 \times 10^{-8}$ |
| SR 42 762 | $4,2 \times 10^{-8}$ |
| SR 42 763 | $1,5 \times 10^{-8}$ |
| SR 42 764 | $4,2 \times 10^{-8}$ |
| SR 42 764 A | $5,2 \times 10^{-8}$ |

Par ailleurs, les composés selon l'invention possèdent une hydrosolubilité assez élevée, C'est ainsi qu'à pH : 7,4, le composé SR 42 654 est soluble dans l'eau à environ 1 %. Dans les mêmes conditions les composés SR 42406 sous forme de chlorhydrate et SR 43054 B possèdent une solubilité dans l'eau de 1 % et 4 % environ.

Les composés de la présente invention ont une action inhibitrice sur l'activité rénine plasmatique humaine très importante et nettement supérieure à celle du produit naturel : la pepstatine. De plus, les composés selon l'invention sont peu toxiques. Ils peuvent être utilisés dans le traitement de l'hypertension artérielle.

Les peptides de la présente invention peuvent être utilisés en thérapeutique par voie injectable : intraveineuse, intramusculaire ou sous-cutanée. Ils sont utilisés dans un solvant, tel que le sérum physiologique (solution saline isotonique) ou dans un tampon, tel que le tampon phosphate.

Ainsi la présente invention concerne également les compositions pharmaceutiques contenant, à titre de principe actif, un peptide selon l'invention en combinaison avec un véhicule pharmaceutiquement acceptable.

La quantité de principe actif à utiliser varie suivant les effets thérapeutiques recherchés, la gravité de l'affection à traiter et la voie d'administration choisie. Elle doit être déterminée pour chaque patient et est le plus souvent comprise entre 1 et 100 mg de principe actif.

## R E V E N D I C A T I O N S

1. Dérivés peptidiques de formule générale

R1 - Phe - R2 - Sta - R3 - Sta - R4 (I)

dans laquelle Sta désigne le radical statyle dérivant de l'aminoacide Statine, à savoir :

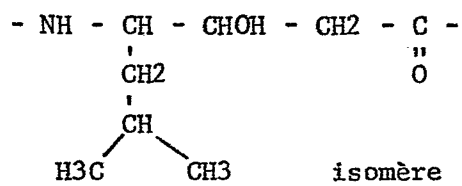

isomère (3S, 4S)

- R1 représente un groupe protecteur de la fonction amine, tel qu'un groupe alcanoyle et en particulier le groupe isovaléryle, un groupe phénylalcanoyle, un groupe aroyle, un groupe arylsulfonyle, un groupe arylalkyl-sulfonyle ou encore un groupe t.butoxycarbonyle (BOC), ou benzyloxycarbonyle (Z), ou adamantyloxycarbonyle (Adoc) ;
- R2 représente le radical dérivé d'un aminoacide choisi parmi la leucine, la norleucine, l'histidine et la norvaline ;
- R3 représente le radical dérivé d'un aminoacide choisi parmi l'alanine, la leucine et l'isoleucine ;
- R4 représente :

- un groupe 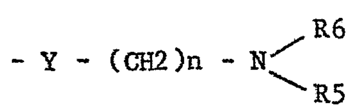

dans lequel : Y est -O- ou -NH-,

n est un entier égal à 2 ou 3,

R5 et 56 pris isolément représentent un groupe alkyle inférieur (1 à 4 C) ou,

R5 et R6 considérés ensemble avec l'atome d'azote auquel ils sont liés forment un cycle, tel que la pyrrolidine, la pipéridine ou la morpholine ;

- un groupe - Y - ($CH_2$) n - $SO_3$ H

où Y et n sont tels que définis ci-dessus

- un groupe $-NH-\underset{\underset{R9}{|}}{\underset{(CH_2)p}{|}}{CH}-COOR8$

dans lequel :

- R8 désigne l'hydrogène ou un groupe alkyle inférieur,
- p représente un nombre entier égal à 1, 2, 3 ou 4,
- R9 désigne un groupe guanidino, amino, $-SO_3H$, ou -COOR8,
- R8 ayant les significations mentionnées ci-dessus ;

- un groupe $-NH-(CH_2)p-\underset{\underset{NH_2}{|}}{CH}-COOR8$

où p et R8 sont tels que définis précédemment,

ainsi que leurs dérivés d'ammonium quaternaires et leurs sels.

2. Dérivés peptidiques selon la revendication 1, caractérisés en ce qu'ils sont sous la forme d'isomères optiques ou de racémiques.

3. Dérivés peptidiques selon l'une des revendications 1 ou 2, caractérisés en ce qu'ils sont les dérivés d'ammonium quaternaires des composés de formule (I) obtenus avec un composé de formule $R_7X$ dans laquelle $R_7$ désigne un groupe alkyle inférieur et X l'anion issu d'un acide minéral ou organique.

4. Procédé pour l'obtention des dérivés peptidiques selon l'une quelconque des revendications 1 à 3, caractérisé en ce que l'on utilise comme produit de départ un ester d'alkyle inférieur de l'acide aminé C terminal sur lequel on couple, étape par étape, les divers acides aminés convenablement protégés, le produit obtenu à chaque étape étant déprotégé, selon des procédés connus, avant d'être soumis à un nouveau couplage, chacune des opérations de couplage étant effectuée en utilisant, soit un ester activé de l'aminoacide à coupler, soit l'aminoacide N protégé en présence de dicyclohexyl-carbodiimide.

5. Procédé selon la revendication 4, caractérisé en ce que la déprotection du produit obtenu est effectuée par hydrogénolyse ou par hydrolyse en milieu acide fort.

6. Procédé pour l'obtention des peptides de formule I selon la revendication 1 dans laquelle $R_4$ n'est pas un aminoacide, caractérisé en ce qu'il consiste à faire réagir un penpapeptide acide de formule :

$$R_1 - Phe - R_2 - Sta - R_3 - Sta - OH$$

dans laquelle $R_1$, $R_2$, $R_3$ sont tels que définis dans la revendication 1, avec un composé de formule $R_4H$ dans laquelle $R_4$ est tel que défini ci-dessus.

7. Procédé selon la revendication 6, caractérisé en ce que, lorsque $R_4$ est le groupe $-Y-(CH_2)_n-N\begin{matrix}\diagup R_6\\ \diagdown R_5\end{matrix}$ tel que défini dans la revendication 1, on obtient les dérivés ammonium quaternaire en faisant réagir lesdits peptides avec un composé de formule $R_7X$ dans laquelle $R_7$ est un groupe alkyle inférieur et X est l'anion issu d'un acide minéral ou organique.

8. Compositions pharmaceutiques caractérisées en ce qu'elles contiennent à titre d'ingrédient actif un dérivé peptidique selon l'une quelconque des revendications 1 à 3.

Office européen des brevets

**0165151**

Numéro de la demande

# RAPPORT DE RECHERCHE EUROPEENNE

EP 85 40 0997

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| X | EP-A-0 081 783 (MERCK)<br>* En entier *<br>--- | 1,2,4,5,8 | C 07 K 5/06<br>A 61 K 37/64 |
| A | EP-A-0 104 964 (INSERM)<br>* En entier *<br>--- | 1,2,8 | |
| A | EP-A-0 077 028 (MERCK)<br>* Page de garde; pages 35,36,42,43 *<br>----- | 1,2,8 | |
| | | | DOMAINES TECHNIQUES RECHERCHES (Int. Cl.4) |
| | | | C 07 K 5/00<br>A 61 K 37/00 |

Le présent rapport de recherche a été établi pour toutes les revendications

| Lieu de la recherche | Date d'achèvement de la recherche | Examinateur |
|---|---|---|
| LA HAYE | 29-08-1985 | RAJIC M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82